# EUROPEAN PATENT APPLICATION

(11) **EP 4 574 194 A1**
(43) Date of publication of application: **25.06.2025**
(21) Application number: 23220147.5
(22) Date of filing: 22.12.2023
(51) Int. Cl.: A61M 25/04, A61M 25/06, A61M 29/02

(54) **A PERCUTANEOUS DELIVERY SYSTEM**

(71) Applicant: PerQHern Limited, 14 Dublin (IE)
(72) Inventor: Keane, David, Dublin, 14 (IE)
(74) Representative: FRKelly

(57) **Abstract**

The present invention is concerned with a percutaneous delivery system, in particular a delivery system for use in delivering medical devices or the like percutaneously to various sites in the human body, the delivery system including an anchoring balloon and an advancing balloon which are arranged to undergo motion relative to one another to advance the delivery system along a lumen within the body.

## Description

### Field of invention

This invention relates to a percutaneous delivery system, in particular a delivery system for use in delivering medical devices or the like percutaneously to the human body.

### Background of the invention

Often procedures in relation to the human body require a delivery system to percutaneously deliver a medical device or substance to a specific site. The delivery system may be used for any number of different purposes and in any number of locations throughout the body. In some instances it is required to deliver a prosthetic mesh for repairing anatomical defects such as hernias and muscle damage or defects.

One instance where percutaneous delivery is used is in treating inguinal hernias, for instance to deliver a suitable repair mesh. The inguinal canal may be relatively flat and the walls of the canal are usually collapsed around their contents. The shape and structure of the inguinal canal may therefore make it difficult for delivery into the canal, especially without risking puncture or damage to the canal. Existing delivery systems include percutaneous sheath systems. An example of which may include a sheath introducer into which a dilator is fully inserted. The dilator and sheath may then be inserted and advanced over a guidewire. The dilator and guidewire may then be removed after full insertion of the sheath. Existing delivery systems may have difficulty in advancing the sheath through the inguinal canal to the site of the hernia due to the shape and structure of the canal.

It is an object of the present invention to provide an improved delivery system for percutaneous delivery which addresses some of the above mentioned problems of the prior art or at least to provide the public with a useful choice.

### Summary of the invention

According to a first aspect of the invention there is provided a percutaneous delivery system comprising a delivery sheath, an anchoring balloon locatable within and displaceable out of a distal end of the delivery sheath, and an advancing balloon located adjacent and distal of the anchoring balloon, wherein the anchoring balloon is reversibly expandable laterally within a body lumen to engage and anchor against a sidewall of the lumen, and the advancing balloon is reversibly expandable longitudinally within the body lumen to advance therethrough.

Preferably, a proximal portion of the advancing balloon is in direct contact with a distal portion of the anchoring balloon such that during longitudinal expansion the advancing balloon bears against the anchoring balloon.

Preferably, the cross-sectional area of the advancing balloon is less than the cross-sectional area of the delivery sheath.

Preferably, the anchoring balloon comprises an outer surface having a high coefficient of friction.

Preferably, the anchoring balloon comprises a roughened outer surface.

Preferably, the anchoring balloon comprises a ribbed outer surface.

Preferably, the anchoring balloon comprises a transversely ribbed outer surface.

Preferably, the anchoring balloon comprises a corrugated ribbed outer surface.

Preferably, the advancing balloon comprises a lubricious coating.

Preferably, the percutaneous delivery system further comprises a steering system adapted to effect deflection of the advancing balloon.

Preferably, the steering system comprising two pulley wires located within longitudinal inner channels in the advancing balloon, wherein the pulley wires are configured to be independently displaced to allow deflection of the advancing balloon during advancement.

Preferably, the advancing balloon comprises two independently expandable sub-balloons within an outer membrane of the advancing balloon that form the steering system.

Preferably, the anchoring balloon is reversibly inflatable.

Preferably, the advancing balloon is reversibly inflatable.

Preferably, the advancing balloon has a substantially circular cross-section.

Preferably, the advancing balloon has a substantially arc-shaped cross-section when deflated and/or when deflated.

Preferably, the arc-shape is approximately 270°.

Preferably, the delivery sheath is transparent.

Preferably, the delivery sheath comprises a soft collar on the sheath tip.

Preferably, the soft collar comprises silicone.

Preferably, the delivery sheath comprises a bevelled tip.

Preferably, the delivery sheath comprises a side aperture.

Preferably, the delivery system further comprises a dilator.

Preferably, the delivery system further comprises a guidewire.

Preferably, the balloons and the delivery sheath are advanced over the guidewire in use.

Preferably, the anchoring balloon is formed from a compliant material.

Preferably, the advancing balloon is formed from a compliant material.

Preferably, the anchoring balloon is formed from a material with a lower compliance than the advancing balloon such that the advancing balloon expands under higher pressure.

Preferably, the delivery system is configured for delivering treatment for inguinal hernias.

According to a second aspect of the invention there is provided a method of percutaneous delivery. Firstly expanding an anchoring balloon located within and displaceable out of a distal end of a delivery sheath. The anchoring balloon being expanded laterally within a body lumen to engage and anchor against a sidewall of the lumen. Secondly expanding an advancing balloon located adjacent and distal of the anchoring balloon. The advancing balloon is expanded longitudinally within the body lumen to advance therethrough. Thirdly retracting the anchoring balloon to disengage and no longer anchor against the sidewall of the lumen. Finally retracting the advancing balloon and advancing the delivery sheath.

Preferably, the method comprises repeating the steps above a plurality of times to advance the delivery system.

As used herein, the term "distal" is intended to mean further away from a user/technician when in use.

As used herein, the term "proximal" is intended to mean closer to a user/technician when in use.

As used herein, the term "lumen" is intended to mean any form of tube-like structure in the body, including canals, vessels. Lumen is also intended to mean any channel that may be created through anatomical tissues in the body either by the present system or another body, for instance a foreign object such as a bullet or the like to be accessed by the system and method of the invention. Lumen can be for example the inguinal canal, gut, oesophagus, small intestine, colon, bile duct, head of the pancreas, parts of the urological system,

### Brief description of the invention

The present invention will now be described with reference to the accompanying drawings, in which:
- Figure 1: illustrates a side view of a delivery system according to a first aspect of the invention in a first configuration;
- Figure 2: illustrates a side view of the delivery system of Figure 1 in a second configuration;
- Figure 3: illustrates a side view of the delivery system of Figure 1 in a third configuration;
- Figure 4: illustrates a side view of the delivery system of Figure 1 in a fourth configuration;
- Figure 5: illustrates a side view of the delivery system of Figure 1 in a fifth configuration;
- Figure 6: illustrates a side view of the delivery system of Figure 3 with a pulley system;
- Figure 7: illustrates a cross-sectional view along the plane A-A of Figure 6;
- Figure 8: illustrates a side view of the delivery system of Figure 3 with two-sub balloons;
- Figure 9: illustrates a cross-sectional view along the plane B-B of Figure 8;
- Figure 10: illustrates a delivery sheath according to an aspect of the invention;
- Figure 11: illustrates a delivery sheath according to another aspect of the invention;
- Figure 12: illustrates a cross-section of an inguinal canal with an advancing balloon according to an aspect of the invention;
- Figure 13: illustrates a delivery sheath with a surgical mesh and an unexpanded third balloon according to an aspect of the invention; and
- Figure 14: illustrates the aspect of Figure 13 with the third balloon expanded.

### Detailed description of the invention

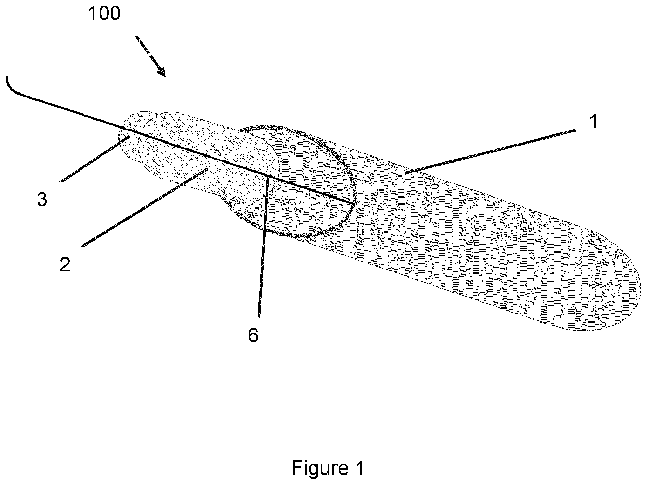

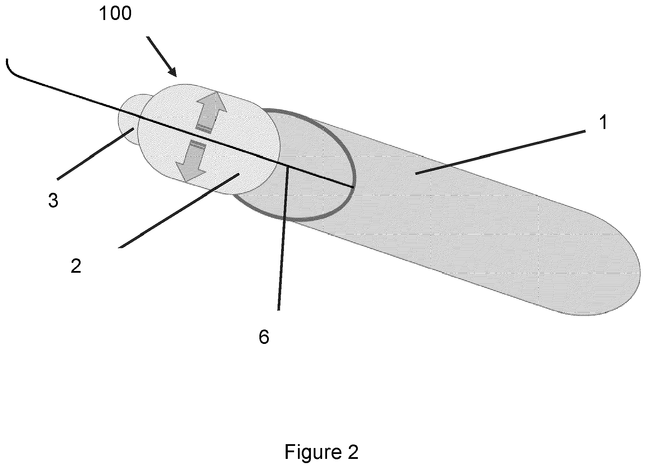

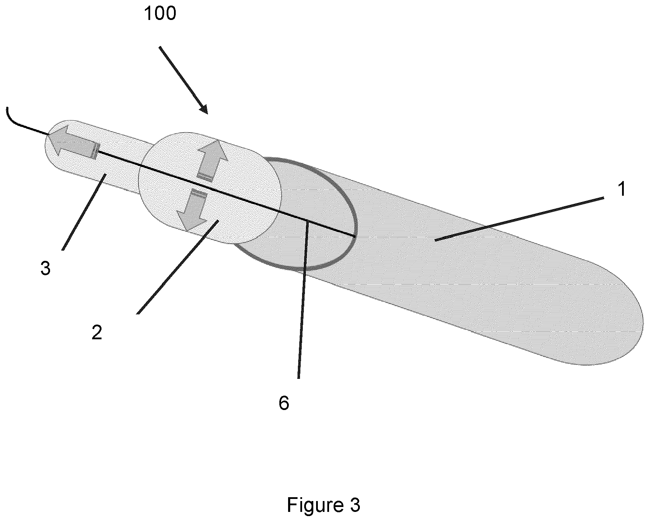

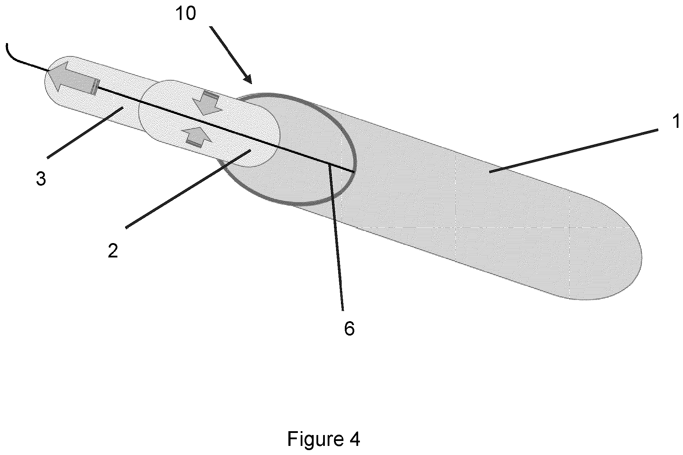

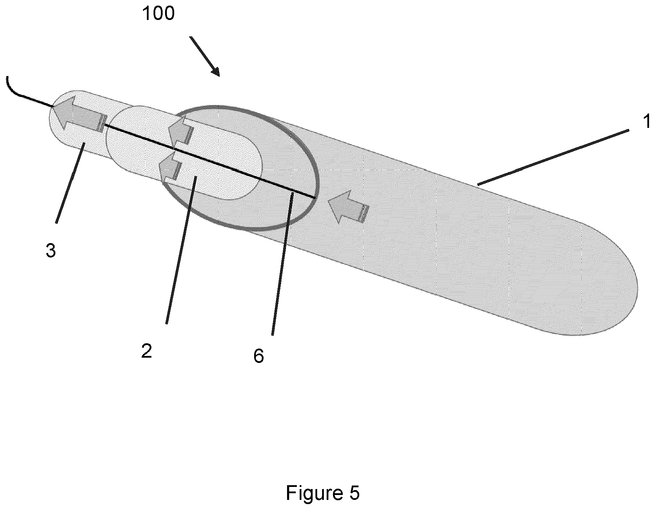

Referring now to Figures 1 to 5 of the accompanying drawings there is illustrated a delivery system, generally indicated by arrow 100. The delivery system 100 is configured for use to deliver, in particular, medical devices such as a hernia repair mesh (not shown) or the like, and/or other substances or medicaments to the human body via percutaneous delivery. The delivery system 100 may be particularly suited for delivery through a body lumen or vessel which may be difficult to advance through, for instance for treating hernias via delivery through the inguinal canal. The delivery system 100 may be used by an interventional radiologist or surgeon under fluoroscopic or ultrasound guidance or internal light emission (translumination). The delivery system 100 may alternatively be used by a surgeon, for instance a minimally invasive surgeon, under sterile conditions with local anaesthetic and conscious sedation. The delivery system 100 may also be used to create a channel or lumen through anatomical tissues, for instance in general surgery to retrieve a foreign object such as a bullet lodged in tissue such as the liver, spleen, brain or other organ, in particular a solid organ without a lateral lumen or potential lumen. The delivery system 100 may be used to create a new and preferably temporary lumen where there was previously no lumen as the delivery system 100 is advanced through the tissue. The methodology by which the delivery system 100 effects forward motion, by a repeating sequence of small movements, ensures minimum disruption to the surrounding tissue when advanced through a solid organ without a pre-existing lumen. The delivery system 100 may be used to deliver treatment to primary tumours or metastatic lesions or to advance scopes and instruments for diagnosis and treatments, for example to perform a colonoscopy or the like. The delivery system 100 may also be used to advance up the urological system for example to retrieve calculi / renal-uretic stones. The delivery system 100 may also have uses in treating animals for instance in veterinary surgery. Thus for example the delivery system 100 may be adapted to be detachably mounted at the forward or distal end of an endoscope (not shown) or similar instrument.

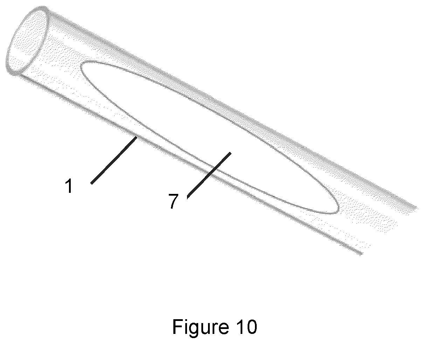

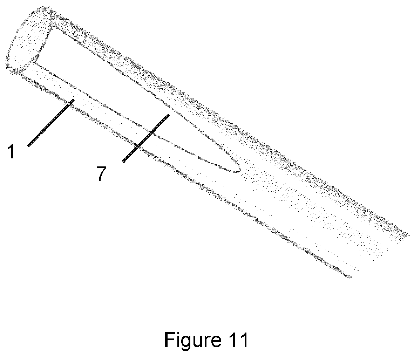

The delivery system 100 comprises a delivery sheath 1. Delivery sheaths are known in the art and generally comprise a tube shape through which medical devices can be supplied to the body. In the example shown in the Figures the delivery sheath 1 is transparent. The delivery sheath 1 may be substantially stiff, semi-rigid, or rigid. In other examples, the delivery sheath 1 may be flexible. The delivery sheath 1 may be non-traumatic, i.e. does minimal or no damage to the tissue as it is advanced. The delivery sheath 1 may comprise a soft collar on the tip of the sheath. The soft collar may comprise a silicone or other relatively soft material such as rubber or a soft plastic. The delivery sheath 1 may also comprise a bevelled or angled tip. The delivery sheath may also comprise one or more apertures 7 to allow any blockages or fluid to pass through from the lumen of the delivery sheath 1 to the surrounding area in use. In some examples, the delivery sheath 1 may comprise a single aperture 7 as shown in Figure 10. The aperture 7 may be oval, circular, rectangular or some other shape in use. In some examples, the aperture 7 may extend to the tip of the delivery sheath 1 as shown in Figure 11. Alternatively or additionally the aperture 7 may be configured to allow surgical access to the walls of the vessel or surrounding tissue. The aperture 7 may therefore be used to seal, cauterize and/or coagulate tissue in the wall of the vessel in use. The apertures 7 may also be configured to allow aspiration of any surrounding blood and inflation or suction/aspiration of carbon dioxide. The delivery sheath 1 may also be configured to deflect. The delivery sheath 1 may for example comprise pulley wires (not shown) that can be pulled to allow either angular changes in direction of the delivery sheath 1 or more gradual rounded changes in direction of the delivery sheath 1. In some examples, the delivery sheath 1 may be pre-curved, i.e. be formed with a fixed curved shape.

The delivery system 100 further comprises an anchoring balloon 2. The anchoring balloon 2 may be located within a distal end of the delivery sheath 1. In other examples the anchoring balloon 2 may be located adjacent to and in contact with the distal end of the delivery sheath 1. The anchoring balloon 2 may be displaceable out of the distal end of the delivery sheath 1. The anchoring balloon 2 may be reversibly expandable laterally within a body lumen such as the inguinal canal in order to engage and anchor against a sidewall of the lumen. The anchoring balloon 2 may expand in a direction towards the sidewall of the lumen. The anchoring balloon 2 may expand predominantly in the lateral direction with minimal expansion in the longitudinal direction. The anchoring balloon 2 may comprise an outer surface having a high coefficient of friction. The anchoring balloon 2 in some examples may have a roughened outer surface. In some examples, the anchoring balloon 2 may comprise one or more ribs or corrugations on the outer surface. The ribs or corrugations may be located transversely along the outer surface such that they assist in preventing longitudinal movement of the anchoring balloon 2 within the lumen. In other examples the anchoring balloon 2 may have one or more projections along the outer surface to assist in anchoring the balloon 2 with respect to the lumen.

The delivery system 100 further comprises an advancing balloon 3. The advancing balloon 3 may be located adjacent to the anchoring balloon 2. The advancing balloon 3 may be located distal of the anchoring balloon 2. The advancing balloon may be reversibly expandable longitudinally within the body lumen to advance therethrough or to create a new lumen as it is advanced. The advancing balloon 3 may expand in a forward direction into the lumen in order to open the lumen which, for example in the case of the inguinal canal, may be flattened due to surrounding abdominal pressure, or may be filled with herniated tissue. The advancing balloon will therefore act to force the herniated tissue back through the canal and into the abdominal cavity, following which a surgical mesh or the like may be deployed to repair the hernia.

The advancing balloon 3 may expand predominantly in the longitudinal direction with minimal expansion in the lateral direction. The advancing balloon 3 may be substantially non-compliant with respect to the maximally inflated diameter but embody some compliance longitudinally. The advancing balloon 3 and the anchoring balloon 2 may primarily expand in directions substantially perpendicular to one another. A proximal portion of the advancing balloon may be in direct contact with a distal portion of the anchoring balloon 2 such that during longitudinal expansion the advancing balloon 3 bears against or abuts the anchoring balloon 2. The advancing balloon 3 may be physically connected to the anchoring balloon along some or all of a longitudinally extending interface there between.

In this way, during deployment, the advancing balloon 3 and anchoring balloon 2 are inseparable to avoid tissue becoming located between the balloons 2, 3. Alternatively the balloons 2, 3 may be arranged to be separable from one another in order to permit tissue to be located between the balloons 2, 3. As the anchoring balloon 2 is engaged and anchored in position the expansion of the advancing balloon 3 will therefore advance the advancing balloon 3 forward or further into the lumen. The advancing balloon 3 may also have a cross-sectional area that is greater than the cross-sectional area of the delivery sheath 1 but less than the cross-sectional area of the anchoring balloon 2, including when the advancing balloon 3 is expanded. The advancing balloon 3 may also comprise a lubricious coating to assist it in advancing through the lumen.

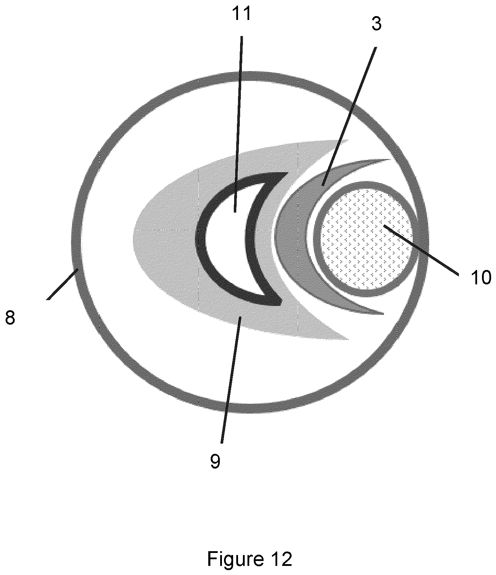

In some examples, the advancing balloon 3 may have a substantially circular cross-section. In other examples as shown in Figure 12, the advancing balloon 3 may have a substantially arc-shaped cross-section when deflated and/or when inflated, i.e. it might be a flat-rolled balloon, and this shape may allow it to better fit between the peritoneal sac and spermatic cord / round ligament of the inguinal canal. In some examples the advancing balloon may be rolled in an arc between 250° and 300°, or approximately 270°. As shown in Figure 12, the advancing balloon 3 is located within the inguinal canal 8. Specifically the advancing balloon 3 is located between the fat or adipose tissue 9 that surrounds the peritoneal sac 11 and the spermatic cord in male patients or the round ligament in female patients 10. The advancing balloon 3 may therefore be used to separate the peritoneal sac and fat from the spermatic cord or round ligament as it is advanced.

In some examples the anchoring balloon 2 and/or the advancing balloon 3 may be reversibly inflatable. In some examples the anchoring balloon 2 and/or the advancing balloon 3 may be inflated by a fluid. In some examples, the fluid may be near freezing, for instance ethanol, to facilitate haemostasis of the surrounding tissue. In other examples, the fluid may be hot, for instance greater than approximately 47°, to facilitate haemostasis of the surrounding tissue. The anchoring balloon 2 and/or the advancing balloon 3 may be formed from a compliant material. In some examples the anchoring balloon 2 is formed from a material with a lower compliance than the advancing balloon 3 such that the advancing balloon 3 expands under higher pressure. This may allow the anchoring balloon 2 to inflate prior to the advancing balloon 3.

In some examples the anchoring balloon 2 and/or the advancing balloon 3 may be substantially transparent. The transparent anchoring balloon 2 and/or the advancing balloon 3 may include optical fibers and optionally a suitable mirror for imaging the surrounding tissue. The transparent anchoring balloon 2 and/or the advancing balloon 3 may also include a diode laser configured to coagulate tissue. The diode laser may also be used through the transparent delivery sheath 1.

In use the delivery system 100 may work by expanding and retracting the anchoring and advancing balloons 2, 3 to advance the delivery sheath 1 through the lumen. The delivery system 100 may move through a number of different configurations during advancement. The first configuration may involve expanding the anchoring balloon 2 laterally such that it engages and anchors against a sidewall of the lumen. The second configuration may involve expanding the advancing balloon 3 longitudinally within the lumen to advance therethrough. The third configuration may involve retracting the anchoring balloon 2 to disengage and no longer anchor against the sidewall of the lumen. Finally the fourth configuration may involve retracting the advancing balloon 3 and advancing the delivery sheath 1 forward. The delivery system 100 may move through these configurations a plurality of times to advance the delivery system 100 along the lumen to the desired location in the body. For each sequence of anchor and then advance, the anchoring balloon 2 may be either partially or fully deflated before advancing the anchoring balloon along the forward track created by the distal advancing balloon 3. The anchoring balloon is then re-inflated or expanded in the new forward position.

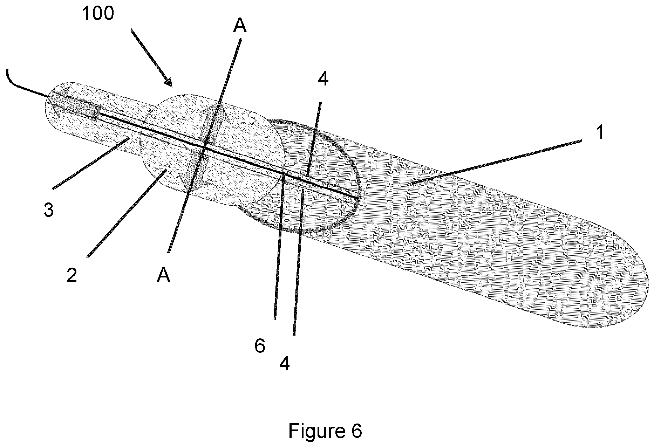

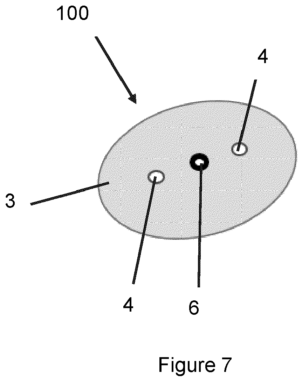

The delivery system 100 may further comprise a steering system adapted to effect deflection of the advancing balloon 3. The steering system may assist in changing the direction of movement of the delivery system 100 to follow contours in the lumen. In the example shown in Figures 6 and 7 the steering system comprises two pulley wires 4. In some forms the steering system may comprise only one pulley wire or in other forms the steering system may comprises more than two pulley wires. The pulley wires 4 may be located within longitudinal inner channels in the advancing balloon 3. The pulley wires 4 may be configured to be individually pulled or advanced to allow deflection of the advancing balloon 3 during advancement. During use one of the pulley wires 4 may be pulled to deflect the advancing balloon 3 in the direction towards the pulley wire. The pulley wires 4 may be located on opposite sides of the advancing balloon 3. The pulley wires 4 may continue through the delivery sheath 1 and may be controlled by the user during use.

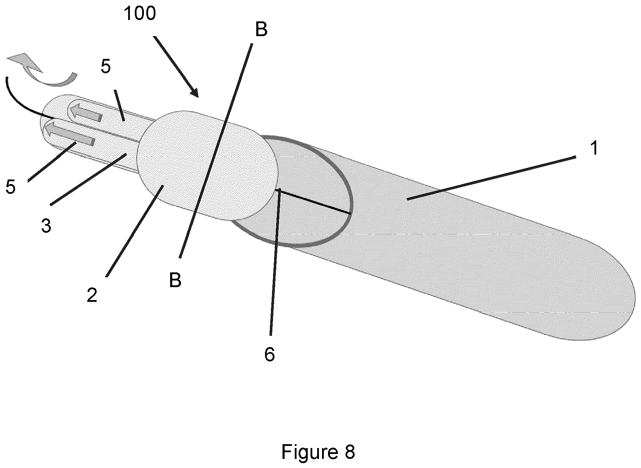

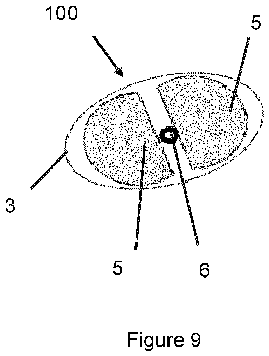

In the example shown in Figures 8 and 9 the steering system may comprise an advancing balloon 3 with two independently expandable sub-balloons 5 located within an outer membrane of the advancing balloon 3. Each of the sub-balloons 5 may be located on opposite sides of the advancing balloon 3. The sub-balloons 5 may be expanded longitudinally. Expansion of one sub-balloon 5 by a greater amount than the other will result in deflection of the advancing balloon 3. Expansion of one sub-balloon 5 more than the other will result in the advancing balloon 3 deflecting in a direction away from the larger sub-balloon 5.

In another example, not shown, the steering system may comprise a central wire with an outer housing that encases the central wire. The outer housing may have a groove that the central wire fits within. The central wire may be configured to be pulled with respect to the outer housing to bend the outer housing and thereby deflect the advancing balloon during advancement. Pulling the central wire may deflect the advancing balloon 3 in a direction towards the central wire. Conversely, the outer housing may be pulled with respect to the central wire to bend the central wire and thereby deflect the advancing balloon 3 during advancement. Pulling the outer housing may deflect the advancing balloon 3 in a direction towards the outer housing.

The delivery system 100 may further comprise a guidewire 6. Guidewire 6 may be advanced first through the body lumen in conventional fashion. The delivery sheath 1 may be advanced over the guidewire 6 in use. The guidewire 6 may be manipulated by the user in use to advance the delivery sheath. The advancing and anchoring balloons 2, 3 may also be advanced over the guidewire 6 in use.

The delivery system 100 may also comprise a dilator, not shown, that is advanced ahead of or distally of the distal end of the delivery sheath 1 to facilitate initial introduction of the delivery system 100. The dilator may be advanced over the guidewire. The dilator may comprise an expandable outer layer that fills the apertures in the delivery sheath 1 in use. The dilator may be at least partially formed from a foam or other soft material. In some examples the dilator may have one or more projections that are raised from the outer surface of the dilator and correspond with the one or more apertures of the delivery sheath 1. The projections may act as a locking mechanism that hold the dilator in place within the delivery sheath 1.

In use, the delivery system 100 may be used to enter the vessel, e.g. inguinal canal, percutaneously under local anaesthesia by using a needle followed by a guidewire 6 and subsequent removal of the needle. The dilator and delivery sheath 1 may then be introduced over the guidewire 6. The dilator and/or guidewire 6 may then be removed. The delivery system 100 may then be advanced along the vessel as described above. An injection of a suitable radiographic contrast may be inserted through the needle under fluoroscopy to confirm the anatomical location of the tip of the delivery sheath 1. An injection of a suitable radiographic contrast may be inserted through the delivery sheath 1 to define the vessel, e.g. inguinal canal, anatomical boundaries as well as other locations, e.g. the location of the inguinal ring. The delivery sheath 1 may also comprise a side arm for use in injecting fluid or gel for instance to reduce the hernia at the start of the procedure. The side arm can also be used to irrigate and aspirate the site of the procedure, for instance by injecting a (prophylactic) antibiotic solution into the site prior to removal of the delivery sheath 1.

When the delivery system 100 is used in the inguinal canal, the tip of the delivery sheath 1 may be directed laterally in the inguinal canal towards the inguinal ring. Furthermore, when the delivery system 100 is used to treat a hernia in the inguinal canal, the delivery sheath 1 may be injected with a foam filler which is injected into the inguinal canal permanently or temporarily to fill the inguinal canal as an additional measure to prevent abdominal viscus re-entering the canal. The foam may be radio-opaque. The foam may be capped beyond the internal inguinal ring either permanently or temporarily. A temporary foam may be bioabsorbable, for instance formed from collagen, or retractable. The foam may also be injected and contained within a rolling membrane, such as a compliant sliding balloon.

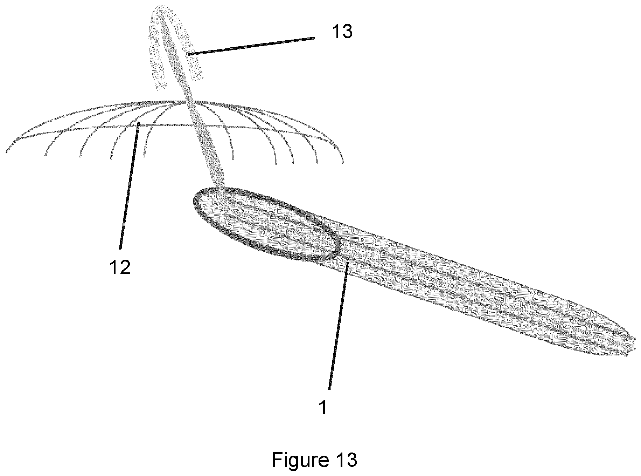

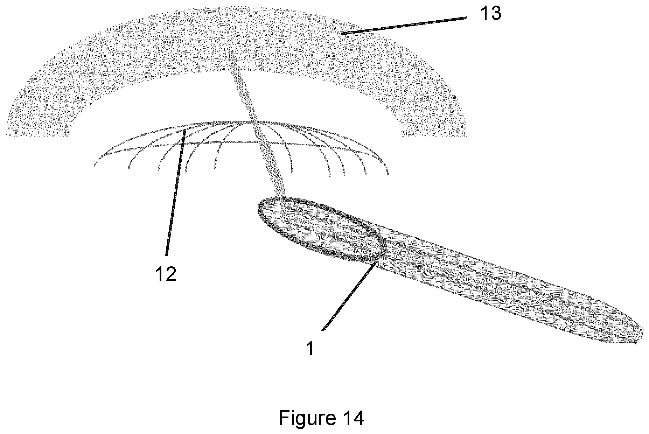

Another feature of the delivery system 100 when used to treat a hernia in the inguinal canal, is that once the delivery sheath 1 has reached the location of the inguinal ring the anchoring balloon 2 and advancing balloon 3 are removed and a surgical mesh is delivered to repair the site of the hernia. In some examples, a plug is deployed through the delivery sheath 1 on the distal side of the internal inguinal ring. In some examples, as shown in Figures 13 and 14, a temporary third balloon 13 may be used to push bowel or abdominal contents, once inside the abdominal cavity, off the site of the hernia, i.e. above the internal inguinal ring, and to clear space for the surgical mesh 12. Carbon dioxide (or other gases) may also be injected through the delivery sheath 1 behind the third balloon 13 to create additional space for the surgical mesh 12. The third balloon 13 may be substantially parasol, dome or umbrella shaped. The third balloon 13 may be expanded to create space for the surgical mesh 12 to expand unimpeded by the bowel or abdominal contents. The surgical mesh 12 may be expanded in a space above the deep inner inguinal ring and underneath the third balloon 13 to treat the hernia.

The mode of operation of the delivery system 100 makes it particularly suited to be advanced through lumens that are comprised of or defined by folds or corrugations of tissue, having irregularly shaped sidewalls, or defining particularly tortuous paths, for example the colon or other sections of the intestinal tract. Such folded or corrugated sidewalls render such lumens difficult to navigate using conventional surgical instruments that are designed to be pushed through the lumen in order to be advanced towards a target site. In lumens such as the colon having sidewalls of folded tissue advancing an instrument such as an endoscope by pushing the instruments through the colon tends to force the tip or head of the instrument into the folds of tissue and the scope can then become caught between folds with the risk that further pushing of the instrument will perforate the sidewall of the lumen.

The delivery system 100 of the invention effects a different mode of advancement as described above, by means of a repeating sequence of steps involving small advances of the anchoring balloon 2 and the advancing balloon 3. When navigating a lumen such as the colon, where the sidewall is folded and is thus relatively extensible in the longitudinal direction due to the excess tissue defined by the folds, the relative movement between the anchoring balloon 2 and advancing balloon 3 draws this folded tissue longitudinally towards and over the balloons 2, 3, effectively smoothing out the folds. In addition, during each sequence, inflating the anchoring balloon 2 radially outward to engage the walls of the lumen will also flatten the folds. In combination these actions allow the system 100 to be advanced without risk of perforating the sidewall by significantly elongating and thus flattening the folds. This flattening is a temporary condition and once the system 100 has been advanced past a section of the colon the tissue will resume the folded state. The delivery system 100 is therefore particularly suited to perform a colonoscopy or similar procedure involving otherwise difficult to navigate bodily lumens.

The delivery system 100 of the invention thus provides a relatively simple and therefore robust means of percutaneous delivery by advancing through an otherwise difficult to navigate bodily lumen in order to permit a surgical device such as a hernia repair mesh to be advanced to a surgical site such as a hernia or to perform a colonoscopy or the like.

The invention is not limited to the embodiments described herein but can be amended or modified without departing from the scope of the present invention.

## Claims

1. A percutaneous delivery system comprising:
a delivery sheath;
an anchoring balloon locatable within and displaceable out of a distal end of the delivery sheath; and
an advancing balloon located adjacent and distal of the anchoring balloon;
wherein the anchoring balloon is reversibly expandable laterally within a body lumen to engage and anchor against a sidewall of the lumen; and
wherein the advancing balloon is reversibly expandable longitudinally within the body lumen to advance therethrough.

2. The percutaneous delivery system of claim 1, wherein a proximal portion of the advancing balloon is in direct contact with a distal portion of the anchoring balloon such that during longitudinal expansion the advancing balloon bears against the anchoring balloon.

3. The percutaneous delivery system of any one of claims 1 to 2, wherein the cross-sectional area of the advancing balloon is less than the cross-sectional area of the delivery sheath.

4. The percutaneous delivery system of any one of claims 1 to 3, wherein the anchoring balloon comprises an outer surface having a high coefficient of friction.

5. The percutaneous delivery system of any one of claims 1 to 4, wherein the anchoring balloon comprises a high friction outer surface.

6. The percutaneous delivery system of claim 5, wherein the anchoring balloon comprises a ribbed outer surface.

7. The percutaneous delivery system of claim 6, wherein the anchoring balloon comprises a transversely ribbed outer surface.

8. The percutaneous delivery system of claim 6, wherein the anchoring balloon comprises a corrugated ribbed outer surface.

9. The percutaneous delivery system of any one of claims 1 to 8, wherein the advancing balloon comprises a lubricious coating.

10. The percutaneous delivery system of any one of claims 1 to 9, wherein the percutaneous delivery system further comprises a steering system adapted to effect deflection of the advancing balloon.

11. The percutaneous delivery system of claim 10, wherein the steering system comprising two pulley wires located within longitudinal inner channels in the advancing balloon, wherein the pulley wires are configured to be pulled to allow deflection of the advancing balloon during advancement.

12. The percutaneous delivery system of claim 10, wherein the steering system comprises: a central wire; and an outer housing that encases the central wire in a groove; wherein the central wire is configured to be pulled to bend the outer housing and thereby deflect the advancing balloon during advancement.

13. The percutaneous delivery system of claim 10, wherein the advancing balloon comprises two independently expandable sub-balloons within an outer membrane of the advancing balloon that form the steering system.

14. The percutaneous delivery system of any one of claims 1 to 13, wherein the anchoring balloon is reversibly inflatable.

15. The percutaneous delivery system of any one of claims 1 to 14, wherein the advancing balloon is reversibly inflatable.

16. The percutaneous delivery system of any one of claims 1 to 15, wherein the advancing balloon has a substantially circular cross-section.

17. The percutaneous delivery system of any one of claims 1 to 15, wherein the advancing balloon has a substantially arc-shaped cross-section.

18. The percutaneous delivery system of claim 17, wherein the arc-shape is approximately 270°.

19. The percutaneous delivery system of any one of claims 1 to 18, wherein the delivery sheath is transparent.

20. The percutaneous delivery system of any one of claims 1 to 19, wherein the delivery sheath comprises a soft collar on the sheath tip.

21. The percutaneous delivery system of claim 20, wherein the soft collar comprises a silicone.

22. The percutaneous delivery system of any one of claims 1 to 21, wherein the delivery sheath comprises a bevelled tip.

23. The percutaneous delivery system of any one of claims 1 to 22, wherein the delivery sheath comprises a side aperture.

24. The percutaneous delivery system of any one of claims 1 to 23, further comprising a dilator.

25. The percutaneous delivery system of any one of claims 1 to 24, further comprising a guidewire.

26. The percutaneous delivery system of claim 25, wherein the balloons and the delivery sheath are advanced over the guidewire in use.

27. The percutaneous delivery system of any one of claims 1 to 26, wherein the anchoring balloon is formed from a compliant material.

28. The percutaneous delivery system of any one of claims 1 to 27, wherein the advancing balloon is formed from a compliant material.

29. The percutaneous delivery system of any one of claims 1 to 28, wherein the anchoring balloon is formed from a material with a lower compliance than the advancing balloon such that the advancing balloon expands under higher pressure.

30. The percutaneous delivery system of any one of claims 1 to 29, wherein the delivery system is configured for delivering treatment for inguinal hernias.

31. A method of percutaneous delivery, the method comprising:
expanding an anchoring balloon located within and displaceable out of a distal end of a delivery sheath, wherein the anchoring balloon is expanded laterally within a body lumen to engage and anchor against a sidewall of the lumen;
expanding an advancing balloon located adjacent and distal of the anchoring balloon, wherein the advancing balloon is expanded longitudinally within the body lumen to advance therethrough;
retracting the anchoring balloon to disengage and no longer anchor against the sidewall of the lumen;
retracting the advancing balloon; and
advancing the delivery sheath.

32. The method of percutaneous delivery of claim 31, the method comprising repeating the steps of claim 31 a plurality of times to advance the delivery system.
